(19) Europäisches Patentamt
European Patent Office
Office européen des brevets

(11) Publication number : 0 499 398 A2

(12) # EUROPEAN PATENT APPLICATION

(21) Application number : 92300970.8

(22) Date of filing : 05.02.92

(51) Int. Cl.[5] : A61K 7/32, B01F 17/00

(30) Priority : 14.02.91 GB 9103192
09.08.91 GB 9117239

(43) Date of publication of application :
19.08.92 Bulletin 92/34

(84) Designated Contracting States :
DE FR GB IT

(71) Applicant : DOW CORNING GmbH
Rheingaustrasse 53, Postfach 130332
W-6200 Wiesbaden 13 (DE)

(72) Inventor : Roidl, Josef Jakob
Fliederweg 14
W-6501 Saulheim (DE)

(74) Representative : Vandamme, Luc Johan Roger
Dow Corning Limited Cardiff Road
Barry South Glamorgan CF6 7YL Wales (GB)

(54) **Antiperspirant composition.**

(57) The specification describes and claims an antiperspirant in the form of a water-in-oil type emulsion comprising an aqueous solution of an astringent antiperspirant agent as a discontinuous phase, a volatile liquid silicone fluid as a continuous phase, an organopolysiloxane-polyoxyalkylene copolymer compatible with the continuous and discontinuous phases of the composition and a surfactant comprising polypropoxylated myristyl alcohol or the propionic acid ester thereof and optionally additives known in the cosmetic art.

The antiperspirant composition according to the invention may be formulated as pump spray, aerosol, roll-on or gel types and has the advantage of exhibiting a substantially reduced whitening effect when applied to skin or fabric.



Jouve, 18, rue Saint-Denis, 75001 PARIS

This invention relates to antiperspirant compositions.

Antiperspirant compositions are well known in the cosmetic art. These compositions are formulated as aerosols, gels, sticks, creams, pump sprays and lotions and comprise an astringent, typically comprising one or more zirconium salts and/or aluminium salts, in various forms such as a dry, impalpable powder, an alcohol solution or an aqueous solution. Of these various forms of astringents the aqueous solution is believed to be the most effective antiperspirant. The present invention is especially concerned with antiperspirant compositions of the so-called dry-feeling type, comprising an emulsion of an aqueous solution of an astringent in a volatile, water-insoluble liquid. Antiperspirant compositions of this type are described for example in G.B. Patent Specification 2 050 162.

Many of the known antiperspirant compositions are in the form of an emulsion and comprise for example, an aqueous solution of an astringent antiperspirant agent as discontinuous phase, a volatile liquid (e.g. a silicone fluid) as a continuous phase, a polydiorganosiloxanepolyoxyalkylene copolymer, an organic surfactant and optionally additives known in the cosmetic art. Whilst such antiperspirant compositions demonstrate many desirable properties, we have found that they tend to give a whitening effect on skin and on fabric to which they have been applied. There is a need for reducing or eliminating such whitening effect.

It is an object of this invention to provide antiperspirant emulsion compositions of the water-in-oil type which have improved efficacy.

We have now found that when polypropoxylated myristyl alcohol or a propionic acid ester thereof is used as an organic emulsifier in such compositions, the compositions when applied to skin or fabrics give a substantially reduced whitening effect to the extent that compositions can be formulated which give substantially no whitening effect.

The present invention provides in one of its aspects an antiperspirant composition in the form of an emulsion comprising an aqueous solution of (a) an astringent antiperspirant agent as discontinuous phase, (b) a volatile liquid silicone fluid as a continuous phase, (c) an organopolysiloxane-polyoxyalkylene copolymer, (d) an organic surfactant and optionally additives known in the cosmetic art characterised in that the organic surfactant (d) comprises polypropoxylated myristyl alcohol or a propionic acid ester thereof.

A composition according to the present invention comprises an astringent antiperspirant agent (a). Examples of well-known astringent antiperspirant agents include the aluminium, hafnium and zirconium salts, such as zirconyl hydroxide halides, zirconium-aluminium complex salts, aluminium chloride, sodium aluminium lactate, basic aluminium halides such as Al2(OH)5Cl, aluminium bromide and the several water, alcohol or glycerine complexes thereof. The amount of agent (a) that is dissolved in water to form the discontinuous phase may vary widely and is not critical. The composition should contain sufficient agent (a) to provide perspiration reduction, although compositions containing less agent (a) are useful as personal care compositions. Preferably the antiperspirant composition comprises approximately 10 to 50 weight percent astringent antiperspirant agent, more preferably 15 to 40 weight percent and most preferably 15 to 30 weight percent. It is desirable to maximize the amount of water in the antiperspirant composition without negating utility, for economic reasons. Depending on the particular agent (a) that is used, the discontinuous phase may comprise from as little as one part by weight agent (a) per five parts by weight water, preferably one part by weight agent per three parts by weight water up to a saturated aqueous solution of the agent (a). Considering economy and efficacy, a particularly useful discontinuous phase comprises an aqueous solution of aluminum chlorhydrate consisting of equal weight portions of water and aluminum chlorohydrate. The aqueous solution (comprising for example from 25 to 50% by weight of the astringent antiperspirant agent) preferably provides from about 30 to 70 parts, more preferably from 40 to 60 parts by weight of the composition.

A composition according to the invention comprises a volatile liquid silicone fluid (b). Suitable materials have a boiling point of less than 250°C at atmospheric pressure. Preferred materials are methylsiloxane fluids having a viscosity at 25°C of less than 10 millipascal-seconds (mPa.s). To avoid an excessive cooling effect for the user of the compositions of this invention it is preferred that at least a portion of the volatile liquid have a normal boiling point of from 100°C to 200°C. The volatile methylsiloxane fluid (b) has the average unit formula

$$(CH_3)_aSiO_{\frac{(4-a)}{2}}$$

where <u>a</u> has an average value of from 2 to 3 and consists of siloxane units selected from the group consisting of $(CH_3)_3SiO_{1/2}$, $(CH_3)_2SiO_{2/2}$, $CH_3SiO_{3/2}$ and $SiO_{4/2}$ units. Preferably the volatile methylsiloxane fluid consists essentially of dimethylsiloxane units and optionally trimethylsiloxane units. Of particular value as volatile fluid (b) are the cyclic siloxanes of the general formula $[(CH_3)_2SiO]_x$, wherein <u>x</u> is an integer of from 3 to 6. A highly preferred methylsiloxane fluid is a mixture of said cyclic siloxanes wherein a major portion is tetramer (x = 4).

We prefer that these cyclic siloxanes provide about 5 to 40 parts by weight per 100 parts of the composition. In addition to these cyclic siloxanes we prefer to include in the composition about 10 to 25 and preferably 10 to 17 parts by weight per 100 parts of the composition of volatile linear polydimethylsiloxanes of the general formula $(CH_3)_3SiO[(CH_3)_2SiO]_ySi(CH_3)_3$, and their mixtures, wherein y is an integer of from 0 to 4 the linear materials having a viscosity in the range of 0.5 to 1.0 $mm^2/s$. For compositions intended for pump spray or similar application we prefer to employ about 30 to 35 parts by weight of cyclic siloxanes per 100 parts of the composition. For compositions intended for roll-on or similar application we prefer to employ about 5 to 35 parts by weight of cyclic siloxanes per 100 parts of the composition. Methylsiloxane fluids suitable for use as volatile fluid (b) in the compositions of this invention, are well known in the chemical and polymer arts; many are commercially available.

If desired, a minor proportion of volatile paraffin may be included in the composition for example hexane or butane or a mixture thereof.

A composition according to the invention comprises an organopolysiloxane-polyoxyalkylene copolymer (c). The copolymer acts as an emulsifier having compatibility with the continuous and discontinuous phases of the composition. Suitable copolymers contain units of the general formula

$$Y_bSiO_{\left(\frac{4-b}{2}\right)}$$

wherein b = 0 to 3 inclusive and Y denotes an aliphatic or aromatic radical comprising 1 to 25 carbon atoms or a polyoxyalkylene segment connected with a silicon atom through a divalent hydrocarbon or hydrocarbonoxy radical via a silicon-carbon or a silicon-oxygen-carbon bond respectively. Suitable organopolysiloxanepolyoxyalkylene copolymers contain at least one oxyalkylene sequent per molecule having a molecular weight of at least 1000.

Preferred organopolysiloxane-polyoxyalkylene copolymers are those polydiorganosiloxane-polyoxyalkylene copolymers (hereinafter referred to as Copolymer 1) which contain at least one polydiorganosiloxane segment consisting essentially of

$$R_bSiO_{\left(\frac{4-b}{2}\right)}$$

siloxane units wherein b has a value from 0 to 3 inclusive and R denotes a radical selected from methyl, ethyl, vinyl, phenyl and a polyoxyalkylene segment connected with a silicon atom of a siloxane unit through a divalent hydrocarbon or hydrocarbonoxy radical, at least one polyoxyalkylene segment having a molecular weight of at least 1000 and consisting of from 0 to 50 mol percent polyoxypropylene units and from 50 to 100 mol percent of polyoxyethylene units, at least one terminal portion of said polyoxyalkylene segment being bonded to a siloxane unit, any terminal portion of said polyoxyalkylene segment not bonded to said siloxane unit being satisfied by a terminating radical, the weight ratio of organopolysiloxane segments to polyoxyalkylene segments in said Copolymer 1 having a value of from 2 to 8. Each polyoxyalkylene segment may be bonded to a siloxane unit with silicon-oxygen-carbon bonds or with silicon-carbon bonds. Although Copolymer 1 is not soluble in water and is therefore not subjected to vigorous hydrolysis in the compositions of this invention, it is preferred that each polyoxyalkylene unit in Copolymer 1 is bonded to the siloxane unit with a silicon-carbon bond. Copolymer 1 has an average of from 1.9 to 2.1 R radicals for every silicon atom. Suitable siloxane units include $R_3SiO_{1/2}$, $R_2SiO_{2/2}$, $RSiO_{3/2}$, and $SiO_{4/2}$ units taken in such molar amounts so that b has an average value of approximately 2 in the copolymer. Said siloxane units may be arranged in linear, cyclic and/or branched fashion. The R radicals of Copolymer 1 may be any radical selected from the group consisting of methyl, ethyl, vinyl, phenyl and a polyoxyalkylene segment connected with a silicon atom through a divalent hydrocarbon or hydrocarbonoxy radical. At least 95 percent of all R radicals in Copolymer 1 are methyl radicals; preferably there is at least one methyl radical bonded to each silicon atom. Divalent radicals linking a polyoxyalkylene segment with a siloxane unit of a polydiorganosiloxane segment preferably contain no more than six carbon atoms. Examples of such divalent radicals include $-C_mH_{2m}O-$, $-C_mH_{2m}-$ and $-C_mH_{2m}CO_2-$ where m is an integer greater than zero. Copolymer 1 may comprise one or more of said polydiorganosiloxane segments. The number of and average molecular weight of the polydiorganosiloxane segments in Copolymer 1 is determined by the desired weight proportion of said segments in Copolymer 1. Preferably Copolymer 1 comprises one polydiorganosiloxane segment having bonded thereto one or more polyoxyalkylene segments. The polyoxyalkylene segments of Copolymer 1 consist essentially of oxyethylene units of the formula $-CH_2CH_2O-$ alone, or in combination with

oxypropylene units of the formula $-CH_2CH(CH_3)O-$, an average of at least half of the oxyalkylene units in the polyoxyalkylene segments being oxyethylene units. The polyoxyalkylene segments thus correspond to the formula $[-CH_2CH_2O-]_p[-CH_2CH(CH_3)O-]_q$ wherein the oxyalkylene units may be arranged in any suitable fashion such as random, alternating or block. The average values of $\underline{p}$ and $\underline{q}$ are such that $\underline{p}$ is greater than or equal to $\underline{q}$ and the sum of $\underline{p} + \underline{q}$ is sufficient to provide an average molecular weight of at least 1,000 for the polyoxyalkylene segments. Preferably the average molecular weight of the polyoxyalkylene segments has a value of from 1,500 to 5,000. The polyoxyalkylene segments of Copolymer 1 are bonded to siloxane units of said Copolymer 1 by at least one terminal portion of said polyoxyalkylene segment, said bonding being by way of a divalent radical, hereinbefore described. It is to be understood that said bonding may be by both terminal portions of said polyoxyalkylene segment in those copolymers comprising more than one polydiorganosiloxane segment. Any terminal portion of the polyoxyalkylene segment of Copolymer 1 that is not bonded to a siloxane unit is satisfied by a terminating radical. The type of said terminating radical is not critical and may be monovalent, thereby terminating one polyoxyalkylene segment or polyvalent, thereby terminating more than one polyoxyalkylene segment. The terminating radicals are made up of atoms selected from the group consisting of carbon, hydrogen, nitrogen, and oxygen and may be for example hydrogen; hydroxyl; alkyl, such as methyl, ethyl, propyl, butyl; benzyl; aryl, such as phenyl; alkoxy such as methoxy, ethoxy, propoxy, butoxy; benzyloxy; aryloxy, such as phenoxy; alkenyloxy, such as vinyloxy and allyloxy; acyloxy, such as acetoxy, acryloxy and propionoxy and amino such as dimethylamino. The number of and average molecular weights of the units in Copolymer 1 are such that the weight ratio of polydiorganosiloxane segments excluding the oxyalkylene part of the R groups, to polyoxyalkylene segments in Copolymer 1 has a value of from 2/1 to 8/1, preferably from 2.5/1 to 4.0/1, and that Copolymer 1 has a preferential solubility in the volatile silicone fluid, a condition necessary for the formation of stable water-in-oil type emulsions of this invention. The weight ratio of polydiorganosiloxane segments to polyoxyalkylene segments as defined above in Copolymer 1 is calculated on the basis of the total weight of polydiorganosiloxane and the total weight of polyoxyalkylene that is joined in the copolymerisation process. For example, if 100 parts by weight of polydiorganosiloxane is joined completely by an addition process, which utilises silicon-bonded hydrogen radicals, with 20 parts by weight of polyoxyalkylene, said weight ratio of the resulting copolymer has a value of 5. Copolymer 1 may either be a block copolymer or a so-called rake copolymer. In the former polyoxyalkylene segments link polysiloxane segments. In the latter polyoxyalkylene segments are pendant from polysiloxane segments.

A further preferred organopolysiloxanepolyoxyalkylene copolymer (hereinafter referred to as Copolymer 2) contains units of the general formula

$$Y_bSiO_{\frac{(4-b)}{2}}$$

wherein $\underline{b}$ and Y are as hereinbefore described and wherein at least two organopolysiloxane-polyoxyalkylene molecules are crosslinked through a crosslinking radical joined thereto by non-hydrolysable bonds. The crosslinking radical is of the general formula $-CH_2CHR^1-R^2-CHR^1-CH_2$ wherein $R^2$ is an organic or organosilicon group which contains no hydrolysable bonds, is not adversely reactive with the ingredients the emulsifier has to stabilise and does not interfere with the organopolysiloxane formation, $R^1$ is selected from the groups consisting of hydrogen atoms and 1 - 3 carbon aliphatic radicals which can be different for each location.

The organic groups $R^2$ are selected from $C_1$ -$C_{10}$ alkylene radicals and cycloalkylene radicals such as cyclohexylene, divalent aromatic radicals such as p-phenylene or o-phenylene and oxygen-containing radicals such as $-COOCH_2CH_2OOC$ and $-CH_2-O-CH_2-$. A preferred crosslinking radical is a vinyl terminated organosiloxane, the most preferred radical being tetramethyl divinylsiloxane. The preferred embodiment of a crosslinked organosiloxanepolyoxyalkylene copolymer (Copolymer 2) is of the following general formula:

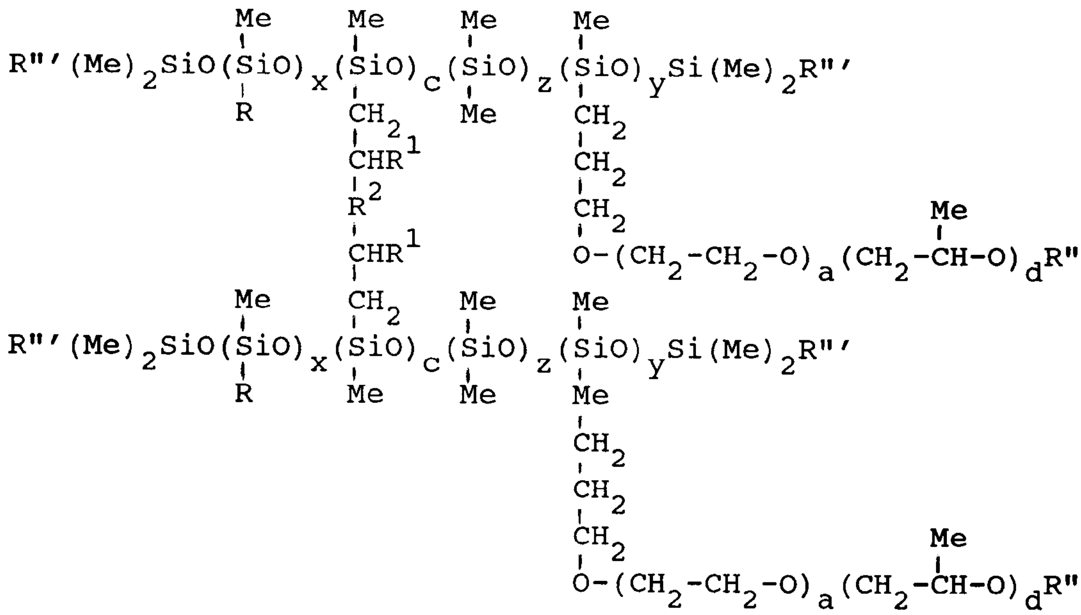

where Me = $CH_3$-, R = 2 to 25 carbon aliphatic radicals, $R^1$ and $R^2$ are as hereinabove defined, R″ = a terminal group which is not adversely reactive with the ingredients the emulsifier is to stabilise and does not interfere with the organopolysiloxane-polyoxyalkylene formation; R‴ = 1 to 25 carbon aliphatic radicals which can be different for each different location, x = 0 to 100, c = 1 to 5, z = 0 to 600, y = 1 to 10, x+y+z = 40, a = 4 to 40 and d = 0 to 40. Such emulsifiers and methods of making them are well known in the art and described in detail in U.S. Patent 4,853,474 dated Ist August 1989 and its equivalent E.P. 0 298 402, each of which is incorporated herein by reference.

Copolymer (c) preferably provides from about 1 to 20 parts, more preferably from 12 to 17 parts by weight of the composition. Copolymers (c) may be prepared according to well-known methods in the silicone art as e.g. described in the polydiorganosiloxane-polyoxyalkylene copolymer art.

In a preferred composition according to the invention Copolymer 1 or Copolymer 2 as hereinbefore described may be used as the sole Copolymer (c) or they may be used in combination such that the weight ratio of Copolymer 1 to Copolymer 2 is of the range of from 5:1 to 10:1.

A composition according to the invention comprises an organic surfactant (d) which comprises polypropoxylated myristyl alcohol or a propionic acid ester thereof. These materials may be used alone or in admixture. They have a unique effect in a composition according to the invention. Not only do they serve to assist in ensuring that the composition is at least substantially stable during storage at room temperature but also contribute significantly to the non-whitening effect on skin and fabrics to which the composition is applied. We prefer to employ from about 1 to about 5 (more preferably 2 to 3) parts by weight of the polypropoxylated myristyl alcohol or a propionic acid ester thereof e.g. those materials which are commercially available as Crodamol® PMP from Croda per 100 parts by weight of the composition. If desired, additional surfactants may be included in a composition according to the invention provided they do not adversely effect the desired properties. Cationic or non-ionic organic surfactant suitable for preparing emulsions of the oil-in-water type and having an HLB value of from 11 to 17, inclusive may be used. Examples of oil-in-water type surfactants include polyethoxylated quaternary ammonium salts and polyoxyethylene fatty amines as cationic surfactants and polyethyleneglycol alkylethers, polyethyleneglycol alkylarylethers, polyglycol esters, sorbitan esters e.g. polyethoxylated sorbitan monolaurate, polyoxyethylene lanolin derivatives and polyethoxylated fatty alcohols as nonionic surfactants. In compositions according to the invention we prefer to include a second organic surfactant, the most preferred material being a polyethoxylated fatty alcohol having from about 12 to about 14 carbon atoms in the alcohol chain and about 3 mole percent of ethoxy units.

A composition according to the invention may further comprise small amounts (for example up to about 5 parts by weight of the composition) of non-essential components which are used in the cosmetic art. Examples of such components include colorants, perfumes, deodorants e.g. zinc ricinoleate and farnesol, viscosity control additives, such as solvents or thickening agents for the continous phase, and non-volatile organopolysiloxanes, such as polydimethylsiloxanes having a viscosity of from 10 to 10,000 millipascal-seconds at 25°C. Well-known adjuvants may be included such as alcohols for gel-formation and solvents to reduce the viscosity of the formulation to less than 100 millipascal-seconds at 25°C for aerosol and pump-spray use.

The compositions of this invention are suitable for use as a lotion preferably packaged and dispersed as a roll-on or pump-spray antiperspirant composition. However, gel and aerosol formulations also may be prepared.

The compositions of this invention may be prepared by mixing the components in any order, but are preferably prepared by preparing a so-called aqueous phase comprising the aqueous solution of an astringent (a) and the organic surfactant and preparing a so-called oily phase comprising the volatile liquid silicone fluid (b) and the polydiorganosiloxane-polyoxyalkylene copolymer (c) and thereafter mixing the so-called aqueous phase with the so-called oily phase. Mixing may be done using standard emulsifying methods.

The present invention provides in another of its aspects an antiperspirant emulsion comprising from 30 to 70 (preferably from 40 to 60) percent by weight of an aqueous solution of an astringent antiperspirant agent as a discontinuous phase, from 5 to 40 (preferably 5 to 35) percent by weight of a volatile cyclic silicone fluid, from 10 to 25 (preferably 12 to 17) percent by weight of a silicone fluid having a viscosity in the range 0.5 to 1.0 centistoke, from 5 to 20 (preferably 10 to 15) parts by weight of an organopolysiloxane-polyoxyalkylene copolymer containing units of the general formula

$$Y_bSiO_{\frac{4-b}{2}}$$

wherein b has a value from 0 to 3 inclusive and Y denotes an aliphatic or aromatic radical comprising 1 to 25 carbon atoms or a polyoxyalkylene segment connected with a silicon atom through a divalent hydrocarbon or hydrocarbonoxy radical, 1 to 5 (preferably 2 to 3) parts by weight of polypropoxylated myristyl alcohol or a propionic acid ester thereof and 0 to 5 parts by weight of additives known in the cosmetic art.

The present invention provides in yet another of its aspects a method of reducing the whitening effect of an antiperspirant composition when applied to skin or fabric by incorporating in that composition a surfactant comprising polypropoxylated myristyl alcohol or the proprionic acid ester thereof.

There now follows a description of example compositions selected to illustrate the invention, example composition C′ being a comparative example and example compositions 1 to 18 being illustrative of the invention. All percentages and parts are by weight; all viscosities were measured at 25°C in mPa.s unless otherwise stated.

The polydiorganosiloxane-polyoxyalkylene copolymer used (designated "Copolymer 1" in the Tables) was prepared from a trimethylsiloxane-endblocked polydimethylsiloxane having a molecular weight of approximately 30,000 and having an average of approximately 4 of its dimethylsiloxane units replaced with methylhydrogensiloxane units, and a random equimolar polyglycol copolymer of ethylene oxide and propylene oxide having an average molecular weight of approximately 2550 and having allyloxy end-groups on one end and acetoxy endgroups on the other end. Two hundred twenty grams of the siloxane, 80.76 grams of the polyglycol and 75.19 grams of isopropanol were mixed and heated to reflux under dry nitrogen in a flask and the resulting solution was catalyzed with 0.15ml of a 1 molar solution of $H_2PtCl_6$ in isopropanol. The reaction mixture was heated at reflux for one hour and then devolatilized at 110°C and 1.33 kilopascals pressure. The copolymer produced had a siloxane/oxyalkylene weight ratio of approximately 2.7/1 and -CH2CH2CH2O- divalent radicals bonding the polyoxyalkylene portion to the polydimethylsiloxane portion by way of a silicon-carbon bond.

The organopolysiloxane-polyoxyalkylene (Copolymer 2 in Table 1) was prepared as described in Example 1 of European Patent Application E.P. 0 298 402 hereby included by reference.

Surfactant 1 was a polypropoxylated myristyl alcohol.

Surfactant 2 was a propionic acid ester of polypropoxylated myristyl alcohol.

Surfactant 3 was a polyethoxylated fatty alcohol having from about 12 to about 14 carbon atoms in the alcohol chain and about 3 mole percent of ethoxy units supplied as Dehydrol L33 by Henkel KGaA.

Surfactant 4 was a polysorbate.

Fluid I was a volatile liquid silicone fluid consisting of a mixture of a major amount of octamethylcyclotetrasiloxane and minor amounts of larger cyclic dimethylsiloxanes.

Fluid II was a volatile liquid silicone fluid consisting of a linear trimethylsilyl endcapped polydimethylsiloxane having a viscosity of 0.65 cSt.

The antiperspirant astringent ingredient used (designated "ACH50" in the Tables) was a 50 % solution of aluminium chlorohydrate in water.

Deodorant was a mixture of 3 parts farnesol and 2 parts zinc ricinoleate.

The example compositions were pump-spray (Examples 1 to 6 and 10 to 15) or roll-on (Examples 7 to 9 and 16 to 18) antiperspirant compositions and were made by preparing aqueous and oily phases and then mixing them together. Each aqueous phase was prepared by mixing Surfactant 4 and a major portion of Surfactant

3, when used, in water with aluminum chlorhydrate in the proportions shown in Table 1. Each oily phase was prepared by dissolving the Surfactant I or II and a minor portion of Surfactant 3, when used, together with the Copolymer in a mixture of Fluid I and Fluid II in the proportions shown in Table 1. The aqueous and oily phases were mixed together for 5 minutes and homogenised for 2 minutes in a Silverson mixer to form the example compositions.

The compositions were charged to glass bottles and fitted with conventional spray nozzles (Examples 1 to 6 and 10 to 15) or conventional roll-on attachments (Examples 7 to 9 and 16 to 18). Prior to use the bottles were shaken to ensure a uniform distribution of the agent (a) throughout the composition. The appearance of the composition was noted as translucent (TU) or white (W).

Various physical properties of the example compositions were determined as follows. Each example composition 1 to 6 and 10 to 15 was sprayed from a bottle towards a sheet of black paper disposed normal to the spray direction and at a distance of 25cm from the nozzle. Each composition was sprayed 3 times to deliver three coats to the paper. The ease with which the composition was applied (i.e. spraying or roll-on) was noted as extremely easy (1), good (2), difficult (3) or not acceptable (4); the spray pattern was noted when wet and after 10 minutes after spraying as good/uniform (GU), uniform broad (UB) or not acceptable (NA). Each example composition 7 to 9 and 16 to 18 was applied to a black sheet of paper by drawing a roll-on applicator across said sheet of paper. The whitening of the paper 30 minutes after spraying or roll-on application was noted as strong (ST), very little (VL) or none (N). The results are reported in Table 2. From these results it can be seen that the compositions which included the propionic acid ester of polypropoxylated myristyl alcohol showed little or no whitening and those which included also polysorbate and/or a polyethoxylated fatty alcohol having from about 12 to about 14 carbon atoms in the alcohol chain and about 3 mole percent of ethoxy units showed the best combination of the properties.

Table 1

Component

| Composition | A | B | C | D | E | F | G | H | I | J | K |
|---|---|---|---|---|---|---|---|---|---|---|---|
| C' | 50 | 7.1 | 33.9 | 0 | 8 | 0 | 0 | 0 | 0 | 1 | 0 |
| 1 | 40 | 5 | 32 | 10 | 10 | 0 | 0 | 2 | 0 | 1 | 0 |
| 2 | 40 | 0 | 31.5 | 15 | 10 | 0 | 0 | 2.5 | 0 | 1 | 0 |
| 3 | 40 | 0 | 32 | 15 | 10 | 0 | 0 | 3.0 | 0 | 0 | 0 |
| 4 | 40 | 0 | 31.2 | 15 | 10 | 0 | 0 | 2.5 | 1.3 | 0 | 0 |
| 5 | 30 | 10 | 30.7 | 15 | 10 | 0 | 0 | 2.5 | 1.3 | 0 | 0.5 |
| 6 | 20 | 20 | 30.7 | 15 | 10 | 0 | 0 | 2.5 | 1.3 | 0 | 0.5 |
| 7 | 40 | 20.5 | 10 | 15 | 10 | 2 | 0 | 2.5 | 0 | 0 | 0 |
| 8 | 40 | 20.5 | 20 | 15 | 0 | 2 | 0 | 2.5 | 0 | 0 | 0 |
| 9 | 40 | 27.5 | 10 | 15 | 0 | 5 | 0 | 2.5 | 0 | 0 | 0 |
| 10 | 40 | 5 | 32 | 10 | 10 | 0 | 2 | 0 | 0 | 1 | 0 |
| 11 | 40 | 0 | 31.5 | 15 | 10 | 0 | 2.5 | 0 | 0 | 1 | 0 |
| 12 | 40 | 0 | 32 | 15 | 10 | 0 | 3.0 | 0 | 0 | 0 | 0 |
| 13 | 40 | 0 | 31.2 | 15 | 10 | 0 | 2.5 | 0 | 1.3 | 0 | 0 |
| 14 | 30 | 10 | 30.7 | 15 | 10 | 0 | 2.5 | 0 | 1.3 | 0 | 0.5 |
| 15 | 20 | 20 | 30.7 | 15 | 10 | 0 | 2.5 | 0 | 1.3 | 0 | 0.5 |
| 16 | 40 | 20.5 | 10 | 15 | 10 | 2 | 2.5 | 0 | 0 | 0 | 0 |
| 17 | 40 | 20.5 | 20 | 15 | 0 | 2 | 2.5 | 0 | 0 | 0 | 0 |
| 18 | 40 | 27.5 | 10 | 15 | 0 | 5 | 2.5 | 0 | 0 | 0 | 0 |

wherein A = ACH 50
B = Water
C = Fluid I
D = Fluid II
E = Copolymer 1
F = Copolymer 2
G = Surfactant 1
H = Surfactant 2
I = Surfactant 3
J = Surfactant 4
K = Deodorant

Table 2

| Property Composition | Appearance | Ease of Application | Spray Pattern | Whitening |
|---|---|---|---|---|
| C' | tU | 2 | Na | St |
| 1 | W | 2 | Ub | Vl |
| 2 | W | 1 | gu | N |
| 3 | W | 1 | gu | Vl |
| 4 | W | 2 | Ub | Vl |
| 5 | tU | 2 | Ub | Vl |
| 6 | tU | 1 | Ub | Vl |
| 7 | W | 3 | Na | Vl |
| 8 | W | 3 | Na | Vl |
| 9 | W | 4 | Na | Vl |
| 10 | W | 2 | Ub | Vl |
| 11 | W | 1 | gu | N |
| 12 | W | 1 | gu | Vl |
| 13 | W | 2 | Ub | Vl |
| 14 | tU | 2 | Ub | Vl |
| 15 | tU | 1 | Ub | Vl |
| 16 | W | 3 | Na | Vl |
| 17 | W | 3 | Na | Vl |
| 18 | W | 4 | Na | Vl |

## Claims

1. An antiperspirant composition in the form of an emulsion comprising an aqueous solution of an astringent antiperspirant agent (a) as discontinuous phase, a volatile liquid silicone fluid (b) as a continuous phase, an organopolysiloxanepolyoxyalkylene copolymer (c), an organic surfactant (d) and optionally additives known in the cosmetic art characterised in that the organic surfactant (d) comprises polypropoxylated myristyl alcohol or a propionic acid ester thereof.

2. An antiperspirant composition according to Claim 1 further characterised in that the polypropoxylated myristyl alcohol or a propionic acid ester thereof provides from 1 to 5 parts by weight of the composition.

3. An antiperspirant composition according to Claim 1 or Claim 2 further characterised in that the volatile liquid silicone fluid comprises a mixture of a volatile cyclic siloxane of the general formula $[(CH_3)_2SiO]_x$, wherein $\underline{x}$ is an integer of from 3 to 6 and a volatile linear polydimethylsiloxane having a viscosity in the range 0.5 to 1.0 $mm^2/s$ at 25°C.

4. An antiperspirant composition according to any one of the preceding claims further characterised in that the volatile liquid silicone fluid is a cyclic siloxane of the general formula $[(CH_3)_2SiO]_x$ wherein $\underline{x}$ has a value of from 3 to 6, provided in an amount of from 5 to 40 parts by weight of the composition.

5. An antiperspirant composition according to Claim 4 further characterised in that the cyclic siloxane provides from 30 to 35 parts by weight of the composition.

**6.** An antiperspirant composition according to Claim 3 further characterised in that the volatile linear polydimethylsiloxane provides from 10 to 25 parts by weight of the composition.

**7.** An antiperspirant composition according to Claim 3 further characterised in that the volatile linear polydimethylsiloxane provides from 10 to 17 parts by weight of the composition.

**8.** An antiperspirant emulsion comprising from 30 to 70 percent by weight of an aqueous solution of an astringent antiperspirant agent (a) as a discontinuous phase, from 5 to 40 percent by weight of a volatile cyclic silicone fluid (b), from 10 to 25 percent by weight of a silicone fluid having a viscosity in the range 0.5 to 1.0 $mm^2/s$ at 25°C, from 1 to 20 percent by weight of a polydiorganosiloxane-polyoxyalkylene copolymer (c) containing at least one polydiorganosiloxane segment consisting essentially of

$$R_bSiO_{\frac{4-b}{2}}$$

siloxane units wherein b has a value from 0 to 3 inclusive, there being an average of 1.9 to 2.1 R radicals for every silicon atom and R denoting a radical selected from methyl, ethyl, vinyl, phenyl and a polyoxyalkylene segment connected with a silicon atom of a siloxane unit through a divalent hydrocarbon or hydrocarbonoxy radical at least one polyoxyalkylene segment having a molecular weight of at least 1000 and consisting of from 0 to 50 mol percent polyoxypropylene units and from 50 to 100 mol percent polyoxyethylene units, at least one terminal portion of said polyoxyalkylene segment being bonded to a siloxane unit, any terminal portion of said polyoxyalkylene segment not bonded to a siloxane unit being satisfied by a terminating radical, the weight ratio of polydiorganosiloxane segments excluding the oxyalkylene part of the R group to polyoxyalkylene segments in said copolymer having a value of from 2/1 to 8/1, from 1 to 5 parts by weight of polypropoxylated myristyl alcohol or a propionic acid ester thereof (d) and 0 to 5 parts by weight of additives known in the cosmetic art.

**9.** An antiperspirant emulsion comprising from 30 to 70 percent by weight of an aqueous solution of an astringent antiperspirant agent (a) as a discontinuous phase, from 5 to 40 percent by weight of a volatile cyclic silicone fluid (b), from 10 to 25 percent by weight of a silicone fluid having a viscosity in the range 0.5 to 1.0 $mm^2/s$, from 1 to 20 percent by weight of an organopolysiloxane-polyoxyalkylene copolymer according to the general formula

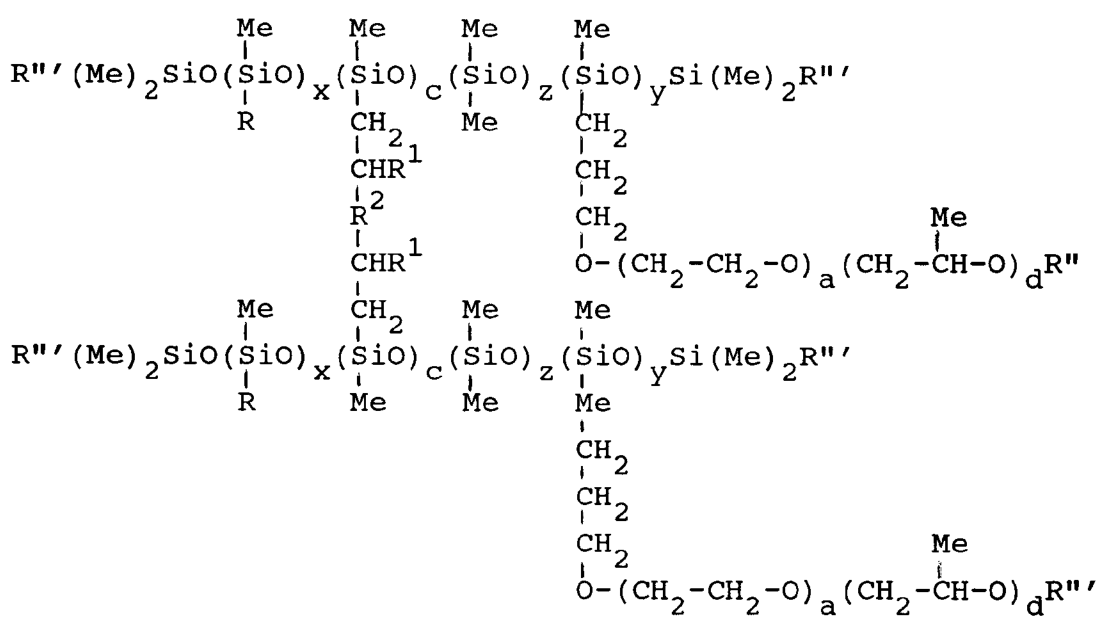

where Me = $CH_3$, R denotes an aliphatic radical having 2 to 25 carbon atoms, $R^2$ = organic or organosi-

loxane group which contains no hydrolysable bonds, is not adversely reactive with the ingredients the emulsifier is to stabilise and does not interfere with the organopolysiloxane-polyoxyalkylene copolymer formation, R″ = a terminal group which is not adversely reactive with the ingredients the emulsifier is to stabilise and does not interfere with the organopolysiloxane-polyoxyalkylene copolymer formation, R‴denotes an aliphatic radical having 1 to 25 carbon atoms, $R^1$ is hydrogen or an aliphatic radical having 1 to 3 carbon atoms, $\underline{x}$ = 0 to 100, $\underline{c}$ = 1 to 5, $\underline{z}$ = 0 to 600, $\underline{y}$ = 1 to 10, $\underline{x+y+z}$ = 40, $\underline{a}$ = 4 to 40 and $\underline{b}$ = 0 to 40, from 1 to 5 parts by weight of polypropoxylated myristyl alcohol or a propionic acid ester thereof (d) and 0 to 5 parts by weight of additives known in the cosmetic art.